# EUROPEAN PATENT SPECIFICATION

(11) **EP 1 785 108 B1**
(45) Date of publication and mention of the grant of the patent: **04.01.2017**
(21) Application number: 06255665.9
(22) Date of filing: 02.11.2006
(51) Int. Cl.: A61F 2/95

(54) **Deployment system for an intraluminal medical device**
Einbringungssystem für eine Intraluminale medizinische Vorrichtung
Systeme de deploiement pour un dispositif medical intraluminal

(30) Priority: 09.11.2005 US 270292
(43) Date of publication of application: 16.05.2007
(73) Proprietor: Cordis Corporation, Miami Lakes, Florida 33014 (US)
(72) Inventor: Buzzard, Jon D., Miramar, FL 33029 (US); Hoo, Bruce A., Miami, FL 33173 (US); Jackson, Karen P., Opa-Locka, FL 33054 (US); Valdes, Francisco, Davie, FL 33314-3741 (US); Widenhouse, Christopher W., Cincinnati, OH 45249 (US)
(74) Representative: Prock, Thomas

(56) References cited:
- WO-A-00/45740
- WO-A-02/056798
- WO-A-2004/066809
- US-A1- 2003 055 378

## Description

The invention relates to a deployment system for an intraluminal medical device. More particularly, the invention, as defined in claim 1, relates to a deployment system having a support member on an inner member of a catheter that helps distribute loads more uniformly througout the intraluminal medical device during loading and delivery.

Percutaneous transluminal angioplasty (PTA) is a therapeutic medical procedure used to increase blood flow through an artery. In this procedure, an angioplasty balloon is inflated within the stenosed vessel, or body passageway, in order to shear and disrupt the wall components of the vessel to obtain an enlarged lumen.

More recently, transluminal prostheses, such as stents, have been used for implantation in blood vessels, biliary ducts, or other similar organs of a patient in order to open, dilate or maintain the patency thereof. Such stents are often referred to as balloon expandable stents, as in US-4,733,665 to Palmaz, as braided or self-expandable stents as in US-4,655,771.

Balloon expandable stents can be impractical for use in some vessels, such as the carotid artery, due to their proximity to the surface of a patient's skin when deployed. Braided stents, on the other hand, pose other disadvantages, such as insufficient radial strength and the risk of unraveling of some of the fibers comprising the braided stent. Other types of self-exapnding stents, such as those comprising shape-memory materials, have thus been devised to address some of these risks. Even self-expanding stents, however, are susceptible to resistive forces during delivery that may cause the stent to undesirably bend, bunch, buckle or break as the stent is attempted to be pushed to its intended treatment site. Reactive forces can accumulate at portions of a stent as a result of such bending or bunching. Non-uniform deployment of the stent can occur as a result.

This tendency is particularly prevalent in newer stent designs that have reduced longitudinal stiffness properties in order to provide greater *in situ* flexibility. Buckling of the stent can further result in undesirable out-of-plane deflections of struts or segments of the stent, which may be particularly prevalent where a series of stents or stent grafts comprise the device. Such deflections risk penetration of the struts or segments into portions of the delivery sheath, or possibly the vessel wall, that can hinder deployment of the stent. Buckling can also lead to undesirable accumulations of reactive forces in portions of the stent that results in non-uniform deployment of the stent as described above. Of course, where resistive forces are so great that breaking of the stent occurs, the stent is no longer usable for its intended purpose. Resistive forces can also damage coatings where a coating stent is used, thereby also rendering the stent unusable. Premature deployment of self-expanding stents also tends to occur in a range of prior art systems.

US-6,302,893 and US-6,077,295 disclose a catheter having an elastomer inner member tube or sleeve on which a stent is positioned and an outer member positioned over the inner member or sleeve. Prior to delivery, the inner member tube or sleeve is heated until it fills and forms attachment projections in the open lattice structure of the stent. The outer member of the catheter retains the self-expanding stent from expanding radially outwardly as the attachment projections of the heated inner member tube or sleeve keeps the stent from undergoing axial or other movement until the outer sleeve of the catheter is withdrawn from over the inner member by relative axial movement of the inner member and the outer member. Upon withdrawal of the outer member, the self-expanding stent expands and extricates itself from the inner member tube or sleeve at an intended treatment site. The heat required to produce the attachment projections of the inner member tube or sleeve of the '893 or '295 patents can weaken bridging, or other portions, of a stent that is to be delivered however. Moreover, the requirement of heat to produce the attachment projections and filling of the open lattice structure of a stent is an additional step that can complicate the delivery of the stent, particularly where insufficient heat is provided rendering the stent susceptible to shifting during delivery as a result of under-developed attachment projections or other open lattice filling functions. Such heat also risks premature, or other, degradation of stent coatings, where the stent to be delivered is provided with such a polymeric, drug or other bio-active agent coating.

Still other delivery systems, such as in US-6,468,298, include preformed protrusions or mechanically retractable grippers that extend from an inner member of a catheter delivery system in order to assist in maintaining a stent in place during delivery thereof. Such mechanically retractable grippers, when extended, engage the struts of a stent during delivery thereof, and when retracted, permit deployment of the stent at the intended treatment site. The mechanics of such grippers can complicate the delivery and deployment of a stent, particularly where the mechanics of such grippers fail. Moreover, such retractable grippers, or such preformed protrusions, must be provided with some degree of precision so as to align properly with the strut configuration of a stent in order to reliably engage, or disengage, the same during delivery.

WO-A-2004/066809 relates to a deployment system for an endoluminal device. It may include an endo-prosthesis mounting member. The endoluminal device is pressed into the mounting member and constrained by a sheath. The mounting member may assist in expansion of the endoluminal device when unconstrained by the sheath.

In view of the above, a need exists for an intraluminal medical device delivery system that more simply and reliably delivers a stent or stent graft to an intended treatment site while more uniformly distributing forces throughout the stent or stent graft during loading and delivery thereof.

Various aspects of the systems and methods of the invention comprise an intraluminal medical device delivery system that simply and reliably delivers a stent or stent graft to an intended treatment site while distributing forces more uniformly throughout the stent or stent graft during loading and delivery.

According to the present invention, there is provided an intraluminal medical device delivery system as defined in appended claim 1. In a preferred embodiment, the intraluminal medical device delivery system comprises a catheter system having an outer member, an inner member, a support member on the inner member of a catheter, and a self-expanding stent or stent graft fitted onto the support member. The stent or stent graft may comprise continuous or discontinous segments forming the stent or stent graft, or combinations thereof. At least one of the inner member and the outer member is axially movable relative to one another and the stent or stent graft is longitudinally supported by the support member as delivery of the stent or stent graft to an intended treatment site occurs.

The support member is comprised of a material having a low modulus of elasticity that increases in modulus when the material is compressed. The stent or stent graft is crimped, i.e., reduced to a non-expanded state, onto the support member so as to mechanically compress the support member. The support member provides increased longitudinal stability to the stent or stent graft by more uniformly distributing resistive forces throughout the stent or stent graft during loading onto the inner member and support member of the delivery system and during delivery thereof to an intended treatment site. Because the stent or stent graft is crimped onto the support member, the stent or stent graft may be more readily moved forward or backward, i.e., towards or away from an intended treatment site, during delivery thereof, which aids re-positioning of the stent or stent graft to effect even more precise placement thereof, when desired or deemed medically preferred. The crimping of the stent or stent graft onto the support member also enables a series of two or more stents or stent grafts, or a series of continuous or discontinous segments of a single stent or stent graft, or a combination thereof, to be moved more readily in unison to effectuate emplacement thereof in an intended treatment site, as desired. The crimping of the stent or stent graft onto the support member in this manner also helps to maintain the stent or stent graft in place during delivery thereof to the intended treatment site, which helps minimize, or ideally eliminates, premature deployment of the stent or stent graft from the delivery catheter.

The support member can be comprised of various materials and morphologies provided it exhibits a soft, flexible, compliant nature. The materials are any known versions of a mechanically compressible material having a modulus of elasticity that increases upon compression, and that recovers its expanded state more slowly than does the stent or stent graft, or segments thereof, upon deployment at the intended treatment site. The morphologies of the support member can be any of a tube, a sleeve, a coating, or a film applied to the surface of the support member and onto which the stent, stent graft, or segments thereof are crimped prior to and during delivery to an intended treatment site.

Generally, the stent, stent graft, or segments thereof, is crimped onto the support member in order to aid stability of the stent, stent graft, or segments thereof, during loading and delivery. In some embodiments, however, portions of the support member protrude through open or accessible areas of the stent or stent graft, or segments thereof, so as to releasably grip the stent or stent graft, or segments thereof. Such gripping by protruding portions of the support member secures the stent, stent graft, or segments thereof, to the inner member of the delivery system even more than otherwise occurs by only crimping until delivery is effected, although crimping alone provides sufficient longitudinal stability to the stent, stent graft or segments thereof, to distribute forces effectively throughout the intraluminal medical device during loading and delivery thereof. Where a stent, stent graft or segments thereof is provided with a coating, the portions of the support structure that protrude through the open or accessible areas of the stent, stent graft, or segments thereof, also provide a barrier to the coating. Such barriers can extend the shelf life of the intraluminal medical device, and helps minimize undesirable or premature delamination of a coating from the device, in addition to the gripping function described above.

In some embodiments, the support member is attached to the inner member of the delivery system using adhesives. In other embodiments, the support member is attached to the inner member by marker bands crimped or swaged over the support member, thereby securing the support member to the inner member. Preferably, one marker band is located at a distal end of the support member, and another marker band is located at a proximal end of the support member. Radiopaque materials may comprise some or all of the marker bands to enhance visualization of the stent, stent graft or segments thereof, and the catheter during delivery and deployment of the intraluminal medical device.

The delivery system may be used in a method of delivering a self expanding intraluminal medical device which generally comprises providing a catheter based delivery system with an outer member, an inner member, a mechanically compressible support member and a self-expanding intraluminal medical device, wherein at least one of the inner member and the outer member are longitudinally movable relative to one another, and the mechanically compressible support member is located along a distal portion of the inner member. The method further comprises loading the intraluminal medical device onto the support member by crimping so as to mechanically compress the support member, restraining the intraluminal medical device in its crimped state and the support member in its compressed state within the outer member, delivering the intraluminal medical device to an intended treatment site by one of pushing the inner member beyond a distal end of the outer member or by withdrawing the outer member while maintaining the inner member in place relative to the intended treatment site, resuming the expanded state of the intraluminal medical device at the intended treatment site and withdrawing the inner member and support member before the support member resumes its fully non-compressed state. The intraluminal medical device may be a stent, a stent graft, segments thereof, or a series of such stents, stent grafts or segments thereof.

The delivery system may also be used in a method of delivering a self-expanding intraluminal medical device to an intended treatment site in a body of a patient, comprising: providing a catheter having an inner member, an outer member, a mechanically compressible support member, the support member located on the inner member and at least one of the inner member and the outer member movable relative to one another; loading and crimping the self-expanding intraluminal medical device onto the support member so as to mechanically compress the support member; restraining the intraluminal medical device in its crimped state and the support member in its compressed state within the outer member; delivering the intraluminal medical device to the intended treatment site by one of pushing the inner member with the support member beyond a distal end of the outer member or withdrawing the outer member while maintaining the inner member with the support member in place relative to the intended treatment site; resuming an expanded state of the intraluminal medical device at the intended treatment site; and withdrawing the inner member and the support member.

The method may further comprise releasably gripping portions of the intraluminal medical device with protruding portions of the support member until delivery of the device is effected at the intended treatment site

The method may further comprise providing a damage minimizing barrier to coatings on the intraluminal medical device by releasably gripping portions of the intraluminal medical device with the protruding portions of the support member.

The support member may be mechanically compressed without the application of heat.

The intraluminal medical device may be fitted onto the support member by one of friction fitting, co-molding, or co-extruding, without additional mechanical members. Repositioning of the intraluminal device may be enabled by the fitting of the intraluminal medical device to the support member.

A modulus of elasticity of the support member increases as the support member is mechanically compressed.

Withdrawing the inner member and support member may occur after or before expansion of the intraluminal medical device has occurred.

The above and other features of the invention, including various novel details of construction and combinations of parts, will now be more particularly described with reference to the accompanying drawings and claims. It will be understood that the various exemplary embodiments of the invention described herein are shown by way of illustration only and not as a limitation thereof. The principles and features of this invention may be employed in various alternative embodiments without departing from the scope of the invention.

Embodiments of the invention will now be described by way of example with reference to the accompanying drawings, in which:
Figure 1 illustrates a conventional catheter delivery system for delivery of self-expanding stents.
Figure 2 illustrates a conventional catheter delivery system having a heat-softened inner member with attachment projections for securing a stent thereto.
Figure 3 illustrates schematically a support member compressed by a stent crimped thereon in accordance with the invention.
Figure 4 further illustrates a stent crimped onto a support member of a catheter delivery system in accordance with the invention.
Figure 5 illustrates the stent of Fig. 4 emerging from the catheter delivery system of Fig. 4 in accordance with the invention.
Figure 6 illustrates the imprint remaining on the support member after delivery, deployment and expansion of the stent has been effected according to the invention.
Figure 7 illustrates another embodiment of a support member of a catheter delivery system in accordance with the invention.
Figure 8 illustrates a stent graft mountable to a delivery system support member by crimping for eventual delivery to an intended treatment site according to the invention.

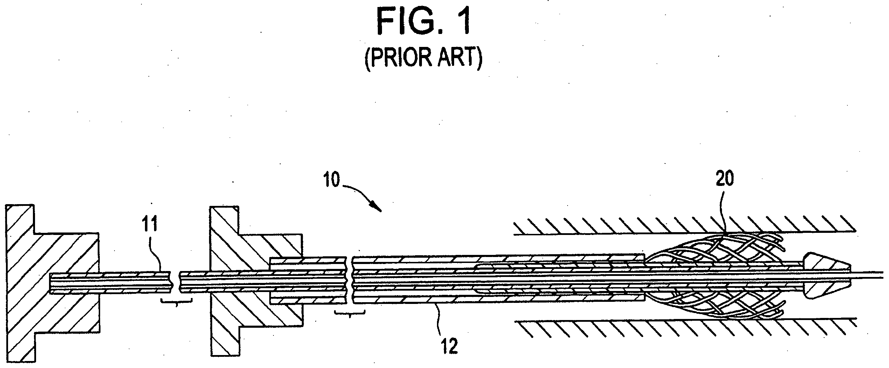

Fig. 1 illustrates a conventional catheter delivery system 10 for delivering a stent 10 into a patient's vasculature, such as into the coronary artery, the carotid artery, the renal artery, peripheral arteries or veins, and the like. As shown in Fig. 1, a self-expanding stent 20 having an open lattice structure is inserted between an inner member 11 and an outer member 12 at a distal end of the catheter delivery system 10. The stent 20 is self-expandable such that the stent 20 expands as the inner member 11 on which the stent is mounted moves beyond the outer member 12. Because the stent 20 is not secured to the inner member 11 prior to delivery, other than by the overlying outer member 12, expansion of the stent occurs rapidly as the stent 20 emerges from the catheter delivery system 10.

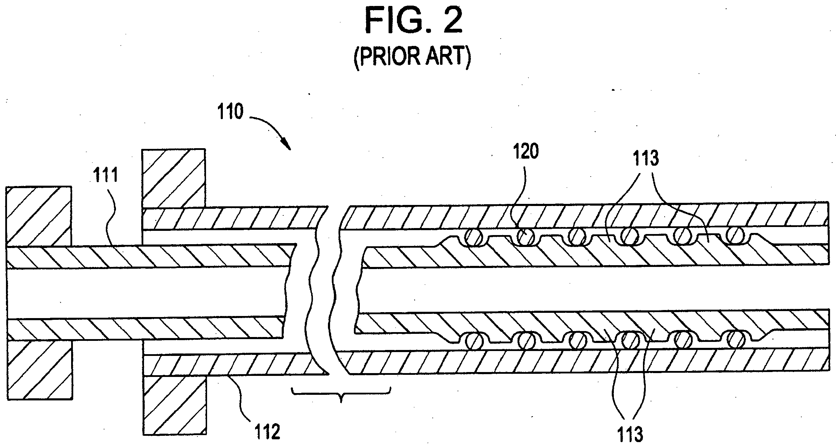

Fig. 2 illustrates a prior art catheter delivery system 110 that is similar to that described above with respect to Fig. 1, except that a distal end of the inner member 111 is made from a polymeric material that is heated to become soft. The outer surface of the heat-softened inner member 111 thus forms attachment projections 113 that fill the open lattice of the stent 120. The attachment projections 113 help to keep the stent 111 from moving axially as delivery of the stent 120 is performed. The attachment projections 113 also help to slow the expansion of the stent 120 after the stent 120 emerges from the constraint of the outer member 112. Better positioning of the stent 120 at an intended treatment site ideally can occur as a result. The heating required to soften the inner member 111 is provided by various methods including by directly heating the inner member111, or by indirectly heating the inner member 111 through the outer member 112.

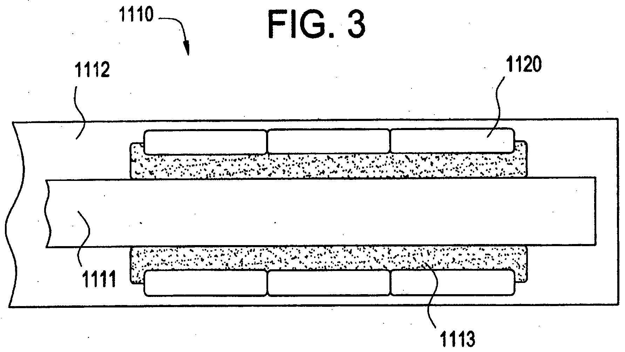

Figs. 3-8 more specifically detail aspects of the intraluminal medical device delivery system according to various embodiments of the invention, wherein the intraluminal medical device is understood to be either a stent, a stent graft, or segments thereof, or a series of such stents, stent grafts, or segments thereof. For descriptive purposes only, the delivery system set forth herein is generally described in terms of a single stent, although any of the various intraluminal medical devices described immediately above are contemplated by this description, unless specifically described otherwise herein. Where stent or stent graft segments are referred to, such are understood to be either continuous, i.e., connected, or discontinous, i.e., disconnected, segments of a stent or stent graft, or a combination thereof, including those segments that are continuous when loaded onto the delivery system wherein some or all of the segments become discontinuous during or after delivery to the intended treatment site.

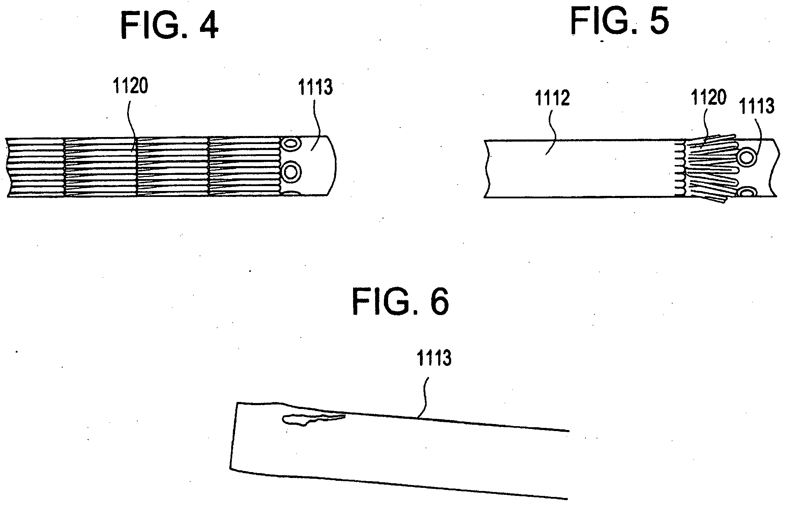

Fig. 3 illustrates schematically a self-expanding stent 1120 crimped onto a distal end of a support member 1111 so as to mechanically compress the support member 1113 according to the invention. No heat is provided to the support member, and no preformed protrusions or other mechanical members are provided on the support member to effectuate the mechanical compression of the support member 1113 or the crimping of the stent 1120 thereon. An outer member 1112 surrounds the inner member 1111 and maintains the stent 1120 and the support member 1113 in their respective crimped and compressed states until delivery is effected. Fig. 4 similarly illustrates a self-expanding stent 1120 crimped onto a distal end of the mechanically compressed support member 1113 of an otherwise conventional catheter delivery system according to the invention, wherein only that portion of the distal end of the inner member of the catheter delivery system is evident in Fig. 4.

Fig. 5 illustrates a self-expanding stent 1120 and inner member 1111 including the support member 1113 emerging from the outer member 1112 of the catheter delivery system 1110. Upon emerging from the distal end of the catheter delivery system 1110, as by pushing the inner member 1111 beyond the distal end of the outer member 1112 or by withdrawing the outer member 1112 from over the inner member while maintaining the inner member 1111 in place relative to an intended treatment site, the stent 1120 expands from its crimped state on the support member 1113, to its expanded state at the intended treatment site.

Fig. 6 illustrates the imprint of the crimped stent 1120 that remains on the support member 1113 after the stent has been delivered and deployed to its intended treatment site. Ideally, the support member 1113 is comprised of a material that takes longer to resume its non-compressed, i.e., expanded, state than it takes the self-expanding stent 1120 to resume its expanded state upon delivery thereof to the intended treatment site. In this way, the expanded stent 1120 overcomes any frictional or other retaining effect of the support member 1113, and the support member 1113 may be withdrawn from the body of the patient so as to minimize damage to or interference with the deployed stent 1120 or vessel in which the stent is emplaced from the support member 1113. Thus, e.g., where the original non-compressed state of the support member 1113 exceeds the original non-compressed state of the stent 1120, then ideally the support member 1113 is withdrawn after the stent 1120 has fully expanded and before the support member 1113 has resumed its fully non-compressed state. Of course, as the artisan will readily appreciate, where the original non-compressed state of the support member 1113 is less than the original non-compressed state of the stent 1120, then withdrawal of the support member 1113 may occur even before full expansion of the stent 1120 at the intended treatment site has occurred without detrimentally impacting the stent 1120 or vessel provided the retaining effect of the support member 1113 is sufficiently overcome by the expansion of the stent 1120. Where the original non-compressed states of the support member 1113 and the stent 1120 are equal or substantially so, then expansion of the stent 1120 to overcome the retaining effects of the support member 1113 is preferable before withdrawal of the support member 1113 occurs.

Referring still to Figs. 3-6, the support member 1113 is comprised of a mechanically compressible material. The mechanically compressible material may be a foam, gel, or other material, including polymers or elastomers, that are normally soft, flexible and mechanically compressible without the assistance of property altering inputs such as heat. In its non-compressed state, the support member preferably has a low modulus of elasticity. When the self-expanding stent 1120 is crimped onto the support member 1113, the material comprising the support member is mechanically compressed and becomes stiffer or more rigid. In this compressed state, the support member 1113 exhibits an increased modulus of elasticity as compared to its modulus of elasticity it the non-compressed state. The increased modulus of elasticity of the support member provides increased longitudinal stability to the stent 1120 during loading of the stent onto the inner member 1111 and support member 1113 of the delivery system 1110 and during delivery of the stent to an intended treatment site. For example, when the stent 1120 is loaded or pushed onto the inner member 1111 and support member 1113 of the delivery system 1110, or when the stent 1120 is otherwise manipulated to be deployed at an intended treatment site, resistive forces experienced by the stent are more uniformly distributed throughout the stent because of the friction fit mounting of the stent 1120 over the support member 1113, rather than such resistive forces being concentrated at a proximal edge of the stent, as occurs in many conventional delivery systems. The more uniform distribution of forces throughout the stent, as a result of its mounting on the support member 1113, helps minimize undesirable bending, buckling, bunching or breaking of the stent during loading and delivery thereof. The support member 1113 is fitted to the inner member 1111 of the delivery system by compression, or friction, fitting a sleeve of the mechanically compressible material onto the inner member 1111, by directly overmolding the mechanically compressible material onto the inner member 1111, or by co-extruding the mechanically compressible material as a layer onto the inner member 1111. Moreover, because the friction fit of the stent 1120 on the support member 1113 ideally secures the stent so well thereon, movement of the stent forwards and backwards, i.e., towards or away from the intended treatment site, can occur with minimal, or ideally no, movement of the stent during such delivery tactics. Repositioning of the stent 1120 to achieve a more precise or desirable emplacement of the stent is also more reliably performed as a result of the friction fitting of the stent to the support member 1113 according to the invention. Thus, the friction fitting of the stent to the support member provides this movement and repositioning capacity without the need for any other attachment members or projections from the support member 1113.

The support member 1113 is comprised of one or more materials that are processable into a mechanically compressible structure that exhibits a higher modulus of elasticity in its compressed state than in its non-compressed state. Such materials include, but are not limited to: polyurethane, polytetrafluoroethylene (in the form of expanded PTFE), silicone, rubbers (such as natural rubbers, EPDM rubbers, epichlorohydrin rubbers, or the like), polyamides, polyimides, fluoropolymers, hydrogels (such as hydroxyethylmethacrylate, polyvinylpyrrolidone, polysulfopropylacrylate, and the like), polyolefins, polyacrylates, methacrylates, or blends and co-polymers thereof, or any other known or later developed material in which its modulus of elasticity increases when in a mechanically compressed state, and that recovers its expanded state more slowly than does the intraluminal medical device, upon deployment of the device at the intended treatment site. The material comprising the support member is also deformable without heat so as to achieve fully the mechanically compressed state thereof solely by the crimping of the intralumnial medical device thereon. Where the material comprising the support member is a foam, the foam may be either an open cell foam or a closed cell foam. The morphologies of the support member can be any of a tube, a sleeve, a coating, or a film, any of which can be applied to the surface of the support member.

As a result of the mechanically compressible materials and morphologies comprising the support member 1113 according to the various embodiments of the invention described herein, stabilization and securement of the stent 1120 on the inner member 1111 and support member 1113 of the catheter delivery system 1110 occurs without heating of the inner member or the support structure, and without providing any additional mechanical members or formations on the surface of the inner member 1111 or support member 1113. Simpler, more reliable, loading and delivery of the stent 1120 to an intended treatment site is thus effected according to the invention.

In some embodiments, when the support member 1113 is mechanically compressed by crimping of the stent 1120 thereon, portions of the support member 1113 protrude between the open lattice-like areas of the stent 1120 so as to releasably grip portions of the stent 1120 with the protruding portions of the support member 1113. Such gripping can provide even more stability to the stent 1120 during loading or delivery thereof, although such gripping is not essential to achieving sufficiently increased stability of the stent to perform loading and delivery thereof to an intended treatment site. Where gripping of the stent 1120 occurs by protruding portions of the support member 1113, such gripping also acts as a barrier that minimizes damage to, and increase the shelf life of, the stent 1120. In particular, such gripping of the stent by the protruding portions of the support member 1113 can also comprise a barrier that minimizes damage to coatings, where such coatings are provided on the stent. Coatings can comprise polymers, drugs or other bio-active agents applied to the stent, as the artisan will readily appreciate, which coatings could more easily delaminate from the stent were the gripping barriers of the protruding portions of the support member not in place. In any event, expansion of the stent 1120 upon delivery to the intended treatment site releases the stent 1120 from the grip of the protruding portions of the support member 1113.

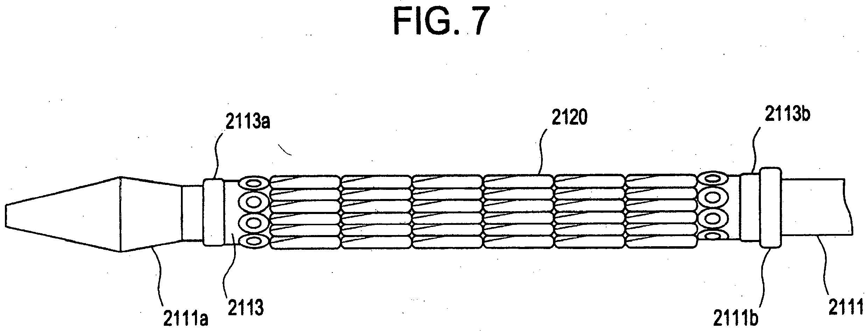

In still other embodiments, as shown in Fig. 7, the support member 2113 is additionally secured to the inner member 2111 of the catheter delivery system. The support member 2113 may be additionally secured to the inner member 2111 by adhesives, as the artisan should readily appreciate, or by marker bands crimped or swaged over ends of the support member. Fig. 7 illustrates, for example, a support member 2113 having a first marker band 2113a securing the distal end of the support member 2113 to the inner member 2111 of the catheter delivery system, and a second marker band 2113b securing the proximal end of the support member 2113 to the inner member 2111 of the catheter delivery system. Of course, more or less marker bands than as shown may be used to secure the support member 2113 to the inner member 2111 of the catheter delivery system. In any event, radiopaque materials may comprise some or all of the marker bands in order to enhance visualization of the stent and catheter during loading, delivery and deployment of the stent to the intended treatement site. As shown also in Fig. 7, the inner member 2111 may further comprise a tapered distal end 2111 a in order to ease insertion and emplacement of the stent to its intended treatment site. The inner member may also comprise a stop member 2111b proximally of the proximal end of the support member 2113 so as to better position the support member 2113 relative to the inner member 2111 of the delivery system.

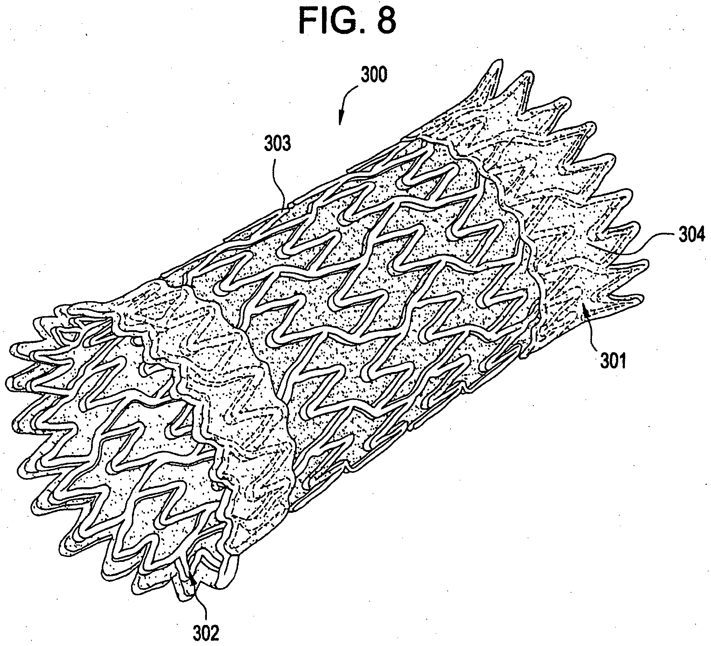

Fig. 8 illustrates one example of a stent graft 300 usable with the various embodiments of the delivery system described herein. As the artisan should appreciate, the stent graft 300 is comprised of a stent 301 having an interior surface 302 and an exterior surface 303. Graft material 304 is provided on either or both surfaces, i.e., the interior surface 302 and the exterior surface 303 of the stent graft 300. The graft material 304 can cover all or only portions of the stent graft 300, as is evident in Fig. 8. Where portions of a support member 1113 or 2113 are intended to grip portions of the stent graft 300, such gripping would most easily occur in areas where graft material 304 has been omitted in the interior surface 302 of the stent graft 300. However, even where graft material 304 is provided over the entire interior surface 302 of the stent graft 300, protruding portions of the support member 1111 or 2114 may releasably grip portions of the stent 301 and the graft material 304 until deployment of the stent graft 300 is achieved as discussed elsewhere herein. Although such stent grafts 300 are most typically employed with respect to treatment of abdominal aortic aneurysms, stent grafts may be delivered for treatment of smaller or peripheral vessels as well using the catheter-based intraluminal medical device delivery system described herein.

In practice, delivery of an intraluminal medical device according to the invention generally comprises providing a catheter based delivery system having an outer member, an inner member, a mechanically compressible support member and a self-expanding intraluminal medical device, wherein at least one of the inner member and the outer member is longitudinally movable relative to one another and the mechanically compressible support member is located along a distal portion of the inner member. The method further comprises loading the intraluminal medical device onto the support member by crimping so as to mechanically compress the support member, restraining the intraluminal medical device in its crimped state and the support member in its compressed state within the outer member, delivering the intraluminal medical device to an intended treatment site by one of pushing the inner member beyond a distal end of the outer member or by withdrawing the outer member while maintaining the inner member in place relative to the intended treatment site, resuming the expanded state of the intraluminal medical device at the intended treatment site and withdrawing the inner member and support member so as to minimize damage to or interference with the device or vessel in which the device is emplaced from the support member as described hereinabove.. The method further comprises releasably gripping portions of the intraluminal medical device with protruding portions of the support member until delivery of the device is effected at the intended treatment site, whereafter expansion of the device releases the device from the grip of the protruding portions of the support member. Releasably gripping portions of the intraluminal medical device in this manner can further provide a barrier that minimizes undesirable, or premature delamination of coatings, where such coatings are provided on the intraluminal medical device. The intraluminal medical device may be a stent or stent graft, or segments thereof. The support member is comprised of materials and morphologies as described hereinabove.

The various exemplary embodiments of the invention as described hereinabove do not limit different embodiments of the systems the invention. The material described herein is not limited to the materials, designs or shapes referenced herein for illustrative purposes only, and may comprise various other materials, designs or shapes suitable for the systems described herein, as should be appreciated by the artisan.

## Claims

1. An intraluminal medical device delivery system comprising:
a catheter having an inner member (1111, 2111) and an outer member (1112), at least one of the inner member (1111,2111) and the outer member (1112) being movable relative to one another;
a support member (1113, 2113) provided on the inner member (111,2111); and
a self-expanding intraluminal medical device (1120) crimped over and mechanically compressing the support member (1113,2113) until delivery of the stent to an intended treatment site is effected;
wherein the support member (1113, 2113) is comprised of a mechanically compressible material having a compressed state and a non-compressed state;
wherein the support member (1113, 2113) resumes some or all of the non-compressed state after the self-expanding intraluminal medical device (1120) has been delivered and expanded at the intended treatment site; and **characterised in that** the mechanically compressible material has a modulus of elasticity that increases when in the compressed state; and
wherein the support member (1113, 2113) recovers its non-compressed state slower than does the intraluminal medical device (1120).

2. The intraluminal medical device delivery system of claim 1, wherein the material comprising the support member (1113, 2113) further comprises a morphology of one of a tube, a sleeve, a coating, and a film, applied to a surface of the support member (1113, 2113).

3. The intraluminal medical device delivery system of claim 2, wherein the material comprising the support member (1113, 2113) further comprises one of a mechanically compressible foam, gel, polymer, or elastomer that deforms without applying heat.

4. The intraluminal medical device delivery system of claim 3, wherein the material comprising the support member (1113,2113) further comprises at least one of polyurethane, polytetrafluoroethylene, expanded PTFE, silicone, natural rubber, EPDM rubber, epichlorohydrin rubber, polyamides, polyimides, fluoropolymers, hydroxyethylmethacrylate, polyvinylpyrrolidone, polysulfopropylacrylate, polyolefins, polyacrylates, methylacrylates, or blends and co-polymers thereof.

5. The intraluminal medical device delivery system of claim 4, wherein the foam is one of an open cell foam and a closed cell foam.

6. The intraluminal medical device delivery system of claim 4, wherein the intraluminal medical device (1120) comprises at least one of a stent, a stent graft, or segments thereof.

7. The intraluminal medical device delivery system of claim 6, wherein the self-expanding intraluminal device (1120) further comprises a coating.

8. The intraluminal medical device delivery system of claim 7, wherein the coating further comprises at least one of a polymeric coating, a drug coating, and a bio-active agent coating.

9. The intraluminal medical device delivery system of claim 6, wherein the support member (1113, 2113), in the mechanically compressed state, further comprises protruding portions releasably gripping portions of the intraluminal medical device (1120).

10. The intraluminal medical device delivery system of claim 6, further comprising a means of securing the support member (1113, 2113) to the inner member (1111,2111).

11. The intraluminal medical device delivery system of claim 10, wherein the means of securing the support member (1113, 2113) to the inner member (1111, 2111) is an adhesive.

12. The intraluminal medical device delivery system of claim 10, wherein the means of securing the support member (1113, 2113) to the inner member (1111, 2111) is at least one marker band (2113a, 2113b) crimped or swaged over the support member (1113, 2113).

13. The intraluminal medical device delivery system of claim 10, wherein the support member (1113, 2113) is directly overmolded onto the inner member (1111, 2111) as the means of securing the support member (1113, 2113) to the inner member (1111, 2111).

14. The intraluminal medical device delivery system of claim 10, wherein the support member (1113, 2113) is co-extruded as a layer onto the inner member (1111, 2111) as the means of securing the support member (1113, 2113) to the inner member (1111, 2111).

15. The intraluminal medical device delivery system of claim 10, wherein the support member (1113, 2113) is compression or friction fitted onto the inner member (1111, 2111) as the means of securing the support member (1113, 2113) to the inner member (1111, 2111).

16. The intraluminal medical device delivery system of claim 12, wherein the at least one marker band (213a, 213b) further comprises a first marker band crimped or swaged over a distal end of the support member and a second marker band crimped or swaged over a proximal end of the support member.

## Patentansprüche

1. Abgabesystem für eine intraluminale medizinische Vorrichtung, umfassend:
einen Katheter mit einem inneren Element (1111, 2111) und einem äußeren Element (1112), wobei zumindest eines vom inneren Element (1111, 2111) und vom äußeren Element (1112) zueinander beweglich sind;
ein Tragelement (1113, 2113), welches auf dem inneren Element (1111, 2111) vorgesehen ist; und
eine selbstexpandierende intraluminale medizinische Vorrichtung (1120), welche auf das Tragelement (1113, 2113) bei mechanischer Kompression desselben umgebördelt ist, bis die Abgabe des Stents an eine vorgesehene Behandlungsstelle ausgeführt ist;
wobei das Tragelement (1113, 2113) ein mechanisch komprimierbares Material umfasst, welches einen komprimierten und einen nicht-komprimierten Zustand aufweist;
wobei das Tragelement (1113, 2113) zum Teil oder vollkommen in seinen nicht-komprimierten Zustand zurückkehrt, nachdem die selbstexpandierende intraluminale medizinische Vorrichtung (1120) abgegeben worden ist und sich an der vorgesehenen Behandlungsstelle expandiert hat; und **dadurch gekennzeichnet, dass** das mechanisch komprimierbare Material ein Elastizitätsmodul aufweist, welches im komprimierten Zustand steigt; und
wobei das Tragelement (1113, 2113) langsamer als die intraluminale medizinische Vorrichtung (1120) in seinen nicht-komprimierten Zustand zurückkehrt.

2. Abgabesystem für eine intraluminale medizinische Vorrichtung nach Anspruch 1, wobei das Material welches das Tragelement (1113, 2113) bildet ferner die Form eines Schlauchs, einer Manschette, einer Beschichtung, oder einer Folie aufweist, welche auf die Oberfläche des Tragelements (1113, 2113) aufgebracht ist.

3. Abgabesystem für eine intraluminale medizinische Vorrichtung nach Anspruch 2, wobei das Material welches das Tragelement (1113, 2113) bildet ferner eines von einem mechanisch komprimierbaren Schaum, einem Gel, einem Polymer oder Elastomer umfasst, welcher sich ohne Wärmeaufbringung verformt.

4. Abgabesystem für eine intraluminale medizinische Vorrichtung nach Anspruch 3, wobei das Material welches das Tragelement (1113, 2113) bildet ferner zumindest einen von Polyurethan, Polytetrafluorethylen, expandiertem PTFE, Silikon, natürlichem Kautschuk, EPDM Kautschuk, Epichlorhydrin Kautschuk, Polyamide, Polyimide, Fluorpolymere, Hydroxyethylmethacrylat, Polyvinylpyrrolidon, Polysulfopropylacrylat, Polyolefine, Polyacrylate, Methylacrylate, oder Mischungen und Copolymeren derselben umfasst.

5. Abgabesystem für eine intraluminale medizinische Vorrichtung nach Anspruch 4, wobei der Schaumstoff ein offenzelliger oder ein geschlossenzelliger Schaumstoff ist.

6. Abgabesystem für eine intraluminale medizinische Vorrichtung nach Anspruch 4, wobei die intraluminale medizinische Vorrichtung (1120) zumindest eines von einem Stent, einem Stentimplantat, oder dessen Segmente umfasst.

7. Abgabesystem für eine intraluminale medizinische Vorrichtung nach Anspruch 6, wobei die intraluminale medizinische Vorrichtung (1120) ferner eine Beschichtung umfasst.

8. Abgabesystem für eine intraluminale medizinische Vorrichtung nach Anspruch 7, wobei die Beschichtung ferner zumindest eines von einer polymeren Beschichtung, einer pharmazeutischen Beschichtung, und einem bio-aktiven Beschichtungsmittel umfasst.

9. Abgabesystem für eine intraluminale medizinische Vorrichtung nach Anspruch 6, wobei das Tragelement (1113, 2113), im mechanisch komprimierten Zustand, ferner vorspringende Abschnitte umfasst, welche Abschnitte der intraluminalen medizinischen Vorrichtung (1120) lösbar greifen.

10. Abgabesystem für eine intraluminale medizinische Vorrichtung nach Anspruch 6, ferner umfassend ein Mittel zum Befestigen des Tragelements (1113, 2113) an das innere Element (1111, 2111).

11. Abgabesystem für eine intraluminale medizinische Vorrichtung nach Anspruch 10, wobei das Mittel zum Befestigen des Tragelements (1113, 2113) an das innere Element (1111, 2111) ein Klebstoff ist.

12. Abgabesystem für eine intraluminale medizinische Vorrichtung nach Anspruch 10, wobei die Mittel zum Befestigen des Tragelements (1113, 2113) an das innere Element (1111, 2111) zumindest aus einem Markierungsband (2113a, 2113b) gebildet sind, welches auf das Tragelement (1113, 2113) umgebördelt oder gestaucht ist.

13. Abgabesystem für eine intraluminale medizinische Vorrichtung nach Anspruch 10, wobei das Tragelement (1113, 2113) auf das innere Element (1111, 2111) direkt aufgeformt ist, als Mittel zum Befestigen des Tragelements (1113, 2113) an das innere Element (1111, 2111).

14. Abgabesystem für eine intraluminale medizinische Vorrichtung nach Anspruch 10, wobei das Tragelement (1113, 2113) auf das innere Element (1111, 2111) coextrudiert ist, als Mittel zum Befestigen des Tragelements (1113, 2113) an das innere Element (1111, 2111).

15. Abgabesystem für eine intraluminale medizinische Vorrichtung nach Anspruch 10, wobei das Tragelement (1113, 2113) auf das innere Element (1111, 2111) durch Kompression oder Reibung aufgesetzt ist, als Mittel zum Befestigen des Tragelements (1113, 2113) an das innere Element (1111, 2111).

16. Abgabesystem für eine intraluminale medizinische Vorrichtung nach Anspruch 12, wobei das zumindest eine Markierungsband (213a, 213b) ferner ein erstes Markierungsband, welches über das distale Ende des Tragelements umgebördelt oder gestaucht ist, und ein zweites Markierungsband, welches über das proximale Ende des Tragelements umgebördelt oder gestaucht ist, umfasst.

## Revendications

1. Système de mise en place d'un dispositif médical intraluminal, comprenant :
un cathéter comportant un élément interne (1111, 2111) et un élément externe (1112), au moins un élément de l'élément interne (1111, 2111) et de l'élément externe (1112) pouvant être déplacé par rapport à l'autre ;
un élément de support (1113) agencé sur l'élément interne (1111, 2111) : et
un dispositif médical intraluminal auto-extensible (1120) serti au-dessus de l'élément de support (1113, 2113) et entrainant une compression mécanique de celui-ci jusqu'à la mise en place du stent au niveau d'un site de traitement voulu ;
dans lequel l'élément de support (1113, 2113) est composé d'un matériau à compressibilité mécanique présentant un état comprimé et un état non comprimé ;
dans lequel l'élément de support (1113, 2113) adopte de nouveau une partie ou l'ensemble de l'état non comprimé après la mise en place du dispositif médical intraluminal auto-extensible (1120) et son extension au niveau du site de traitement ; et **caractérisé en ce que** le matériau à compressibilité mécanique présente un module d'élasticité qui est accru dans l'état comprimé ; et
dans lequel l'élément de support (1113, 2113) récupère son état non comprimé plus lentement que le dispositif médical intraluminal (1120).

2. Système de mise en place d'un dispositif médical intraluminal selon la revendication 1, dans lequel le matériau composant l'élément de support (1113, 2113) comprend en outre une morphologie d'un élément parmi un tube, une gaine, un revêtement ou un film, appliqué sur une surface de l'élément de support (1113, 2113).

3. Système de mise en place d'un dispositif médical intraluminal selon la revendication 2, dans lequel le matériau composant l'élément de support (1113, 2113) comprend en outre une substance parmi une mousse à compressibilité mécanique, un gel, un polymère ou un élastomère, se déformant sans application de chaleur.

4. Système de mise en place d'un dispositif médical intraluminal selon la revendication 3, dans lequel le matériau composant l'élément de support (1113, 2113) comprend en outre au moins une substance parmi un polyuréthane, un polytétrafluoréthylène, du PTFE expansé, du silicone, du caoutchouc naturel, du caoutchouc EPDM, du caoutchouc d'épichlorhydrine, des polyamides, des polyimides, des polymères fluorés, de l'hydroxyéthylmétacrylate, du polyvinylpyrrolidone, du polysulfonepropylacrylate, des polyoléfines, des polyacrylates, des méthylacrylates ou des mélanges et des copolymères de ses substances.

5. Système de mise en place d'un dispositif médical intraluminal selon la revendication 4, dans lequel la mousse est une mousse à alvéoles ouverts ou une mousse à alvéoles fermés.

6. Système de mise en place d'un dispositif intraluminal selon la revendication 4, dans lequel le dispositif médical intraluminal (1120) comprend au moins un stent, une endoprothèse ou des segments de ceux-ci.

7. Système de mise en place d'un dispositif médical intraluminal selon la revendication 6, dans lequel le dispositif médical auto-extensible (1120) comprend en outre un revêtement.

8. Système de mise en place d'un dispositif médical intraluminal selon la revendication 7, dans lequel le revêtement comprend en outre au moins un revêtement parmi un revêtement polymère, un revêtement de médicament ou un revêtement à agent bioactif

9. Système de mise en place d'un dispositif médical intraluminal selon la revendication 6, dans lequel l'élément de support (1113, 2113) comprend en outre, dans l'état à compression mécanique, des parties en saillie saisissant de manière amovible des parties du dispositif médical intraluminal (1120).

10. Système de mise en place d'un dispositif médical intraluminal selon la revendication 6, comprenant en outre un moyen pour fixer l'élément de support (1113, 2113) sur l'élément interne (1111, 2111).

11. Système de mise en place d'un dispositif médical intraluminal selon la revendication 10, dans lequel le moyen servant à fixer l'élément de support (1113,2113) sur l'élément interne (1111, 2111) est un adhésif.

12. Système de mise en place d'un dispositif médical intraluminal selon la revendication 10, dans lequel le moyen servant à fixer l'élément de support (11132, 2113) sur l'élément interne (1111, 2111) est constitué par au moins une bande de marquage (2113a, 2113b) sertie ou estampée au-dessus de l'élément de support (113, 2113).

13. Système de mise en place d'un dispositif médical intraluminal selon la revendication 10, dans lequel l'élément de support (1113, 2113) est directement surmoulé sur l'élément interne (1111, 2111), pour constituer le moyen de fixation de l'élément de support (1113, 2113) sur l'élément interne (1111, 2111).

14. Système de mise en place d'un dispositif médical intraluminal selon la revendication 10, dans lequel l'élément de support (1113, 2113) est co-extrudé sous forme d'une couche sur l'élément interne (1111, 2111) pour constituer l'élément de fixation de l'élément de support (1113, 2113) sur l'élément interne (1111, 2111).

15. Système de mise en place d'un dispositif médical intraluminal selon la revendication 10, dans lequel l'élément de support (1113, 2113) est ajusté par compression ou friction sur l'élément interne (1111, 2111) pour constituer le moyen de fixation de l'élément de support (1113, 2113) sur l'élément interne (1111, 2111).

16. Système de mise en place d'un dispositif médical intraluminal selon la revendication 12, dans lequel la au moins une bande de marquage (213a, 213b) comprend en outre une première bande de marquage sertie ou estampée au-dessus d'une extrémité distale de l'élément de support, et une deuxième bande de marquage sertie ou estampée au-dessus d'une extrémité proximale de l'élément de support.
